# EUROPÄISCHE PATENTSCHRIFT

(11) **EP 0 998 245 B1**
(45) Veröffentlichungstag und Bekanntmachung des Hinweises auf die Patenterteilung: **18.12.2002**
(21) Anmeldenummer: 98941383.6
(22) Anmeldetag: 20.07.1998
(51) Int. Cl.: A61F 2/34

(54) **PFANNE FÜR EINE GELENKENDOPROTHESE**
CUP FOR A KNEE-JOINT PROSTHESIS
CUPULE DESTINEE A UNE ENDOPROTHESE D'ARTICULATION

(30) Priorität: 22.07.1997 DE 19731442
(43) Veröffentlichungstag der Anmeldung: 10.05.2000
(73) Patentinhaber: PLUS ENDOPROTHETIK AG, 6343 Rotkreuz (CH)
(72) Erfinder: SCHMOTZER, Hans, CH-5000 Aarau (CH)
(74) Vertreter: Popp, Eugen, Dr.
(86) Internationale Anmeldenummer: EP9804528
(87) Internationale Veröffentlichungsnummer: WO99004732

(56) Entgegenhaltungen:
- EP-A- 0 640 325
- WO-A-94/23670
- WO-A-97/16138
- WO-A-97/19656
- DE-A- 4 408 527
- FR-A- 2 715 556
- GB-A- 2 159 416
- US-A- 4 666 448
- US-A- 4 892 549

## Beschreibung

Die Erfindung betrifft eine metallene Pfanne für eine Gelenkendoprothese, die schalenartig ausgebildet ist und eine konvexe Außenfläche und konkave Innenfläche aufweist. Derartige Pfannen sind allgemein bekannt und dienen zur Aufnahme von sogenannten Inlays aus Polyethylen, Keramik oder dergleichen humanverträglichem Material. Pfannen dieser Art werden oft als Bestandteil eines künstlichen Hüftgelenks benutzt. In der Regel bestehen sie aus Titan oder einer Titanlegierung. Bei zementloser Implantation ist es des weiteren von Vorteil, wenn die Außenfläche aufgerauht oder porös ausgebildet ist, um das An- und Einwachsen des Knochens an bzw. in die Poren der Pfanne hinein zu begünstigen.

Die Merkmale des Oberbegriffs des Anspruchs 1 sind aus der Druckschrift DE-A-44 08 527 bekannt.

Aus der EP 0 380 055 B1 ist es bekannt, eine halbkugelförmige Pfanne im Bereich ihres Pols gegenüber der Kreis- bzw. Kugelform mit einer Abflachung zu versehen.

In der WO 94/23670 ist vorgeschlagen, daß die Außenfläche einer Hüftgelenkpfanne einen ellipsoidalen Bereich umfaßt.

Die beiden vorbekannten Konstruktionen sollen einen im Vergleich zur reinen Halbkugelschale verbesserten Halt im Knochen gewährleisten. Versuche haben jedoch gezeigt, daß dieses Ziel mit einer Geometrie gemäß der EP 0 380 055 B1 nur unzureichend erhalten wird. Aufgabe dieser bekannten Konstruktion ist es daher auch nur, die Polfläche zurückzunehmen, um zu vermeiden, daß bei Belastung der Knochen im natürlichen Pfannenlager durch Druck auf die Polfläche die Pfanne aus ihrer Preßverankerung im natürlichen Pfannenlager herausdrückt. Durch die Rücknahme der Polfläche soll ein Kontakt zwischen dieser und dem Knochen auch unter starker Belastung sicher vermieden werden. Der Halt der Pfanne im natürlichen Pfannenlager wird dann ausschließlich durch einen Preßsitz im Äquatorialbereich der Pfanne sichergestellt. Dieser Halt reicht sehr häufig nicht aus. In Kenntnis dieser Problematik ist in der WO 94/23670 vorgeschlagen, die Außenfläche einer Hüftgelenkpfanne ellipsoidal auszubilden. Dadurch wird zum einen eine Rücknahme der Polfläche mit den oben geschilderten Vorteilen erreicht; zum anderen wird eine vergrößerte Preßsitzfläche zwischen Pfanne und natürlichem Pfannenlager erhalten. Nachteilig ist jedoch der Herstellungsaufwand für eine ellipsoidale Oberfläche, die eine herstellungstechnisch komplexe Raumfläche darstellt.

Der vorliegenden Erfindung liegt daher die Aufgabe zugrunde, eine Pfanne der eingangs genannten Art zu schaffen, die sich zum einen durch einen verbesserten Preßsitz innerhalb des natürlichen Pfannenlagers und zum anderen durch erheblich niedrigeren Herstellungsaufwand im Vergleich zu der Konstruktion gemäß der WO 94/23670 auszeichnet.

Diese Aufgabe wird erfindungsgemäß durch die Merkmale des Anspruchs 1 gelöst. Die Kugelsegementflächen gemäß Anspruch 1 lassen sich im Vergleich zu einer ellipsoidalen Fläche erheblich einfacher herstellen, und zwar aufgrund ihrer Rotationssymmetrie. Verankerungstechnisch bietet die erfindungsgemäße Konstruktion etwa dieselben Vorteile wie die Konstruktion mit ellipsoidalen Flächenbereichen.

Je mehr Kugelsegementflächen die Außenfläche der Pfanne definieren, desto weniger abrupt sind auch die Übergänge zwischen den jeweils benachbarten Umfangsflächenabschnitten mit der Folge, daß auch dadurch bedingte Spannungsspitzen im Knochen entsprechend reduziert sind. Diese Spannungsspitzen beeinflussen die Verankerungsfestigkeit der Pfanne im Knochen nachteilig. Daher gilt es, solche Spannungsspitzen, d. h. abrupten Flächenübergänge zu vermeiden. Dies wird erfindungsgemäß erreicht.

Vorteilhafte konstruktive Details der Erfindung sind in den Unteransprüchen beschrieben.

Um die Pfanne im Polbereich abzuflachen, liegt das Zentrum der peripheren bzw. äquatorialen Kugelsegmentfläche näher am Pol der Pfanne als das Zentrum der polnahen Kugelsegmentfläche. insbesondere als das Zentrum der Polfläche selbst.

Um die Pfanne rotationsfest im natürlichen Pfannenlager zu halten, kann es vorteilhaft sein, die äquatoriale Kugelsegmentfläche mit polargerichteten Rippen oder dergleichen Erhebungen zu versehen.

Nachstehend wird eine Ausführungsform einer erfindungsgemäßen Hüftgelenkpfanne anhand der beigefügten Zeichnung näher erläutert.

Es zeigen:
- Fig. 1: eine sogenannte Triple-Radius-Pfanne in Seitenansicht unter Darstellung der verschiedenen Kugelsegment-Bereiche im Verhältnis zu einer Halbkugelschale in Seitenansicht; und
- Fig. 2: eine konkrete Ausführungsform einer Triple-Radius-Pfanne gemäß Fig. 1 in perspektivischer Schrägansicht von unten bzw. außen.

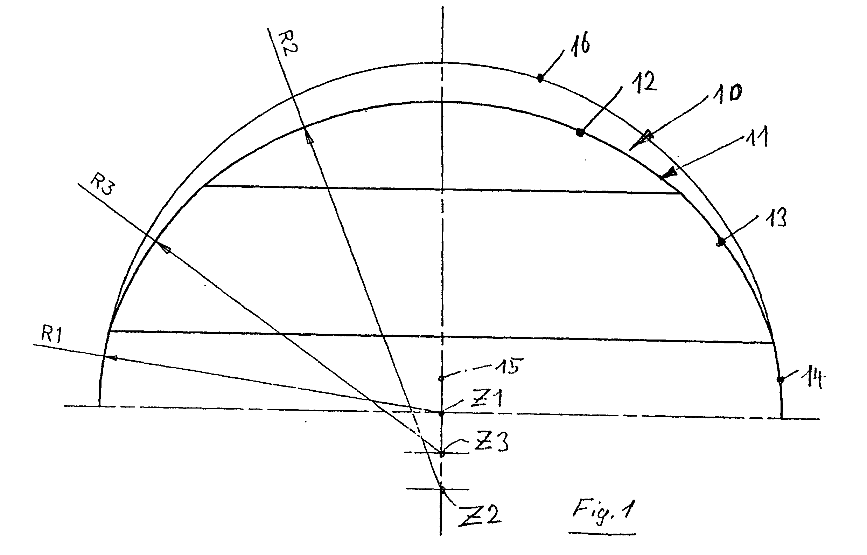

Die Fig. 1 zeigt eine Pfanne 10 mit einer konvexen Außenfläche 11, die definiert ist durch drei sich über den Umfang erstreckende Kugelsegmentflächen 12, 13, 14 mit jeweils unterschiedlichem Radius. Die drei Kugelsegmentflächen 12, 13, 14 weisen eine gemeinsame Rotationsachse 15 auf. Auf dieser Rotationsachse 15 liegen die Zentren Z1, Z2 und Z3 der einzelnen Kugelsegmentflächen 14, 12 und 13 mit den Radien R1, R2 und R3, wobei der Radius R3 der medialen Kugelsegmentfläche 13 kleiner ist als der Radius R2 der polaren Kugelsegmentfläche 12, aber größer ist als der Radius R1 der äquatorialen Kugelsegmentfläche 14. Durch diese Konfiguration und Auswahl der Kugelradien ergibt sich im Bereich des Pols der Pfanne 10 eine Abflachung im Verhältnis zu einer reinen Halbkugelschale, die in Fig. 1 mit dem Halbkreisbogen 16 angedeutet ist.

Die Fig. 1 läßt des weiteren erkennen, daß bei der dargestellten Ausführungsform die äquatoriale Kugelsegmentfläche 14 sowie polare Kugelsegmentfläche 12 jeweils eine geringere Breite aufweisen als die dazwischen liegende bzw. mediale Kugelsegmentfläche 13. Konkret weisen die äquatoriale und polare Kugelsegmentfläche etwa die halbe Breite der medialen Kugelsegmentfläche 13 auf. Diese Ausführungsform gewährleistet eine bessere Anpassung der Pfanne 10 an das natürliche Pfannenlager. Damit wird auch die Anwachsung des Knochens an der Pfanne begünstigt mit der Folge, däß der Pfannensitz zunehmend erhöht wird.

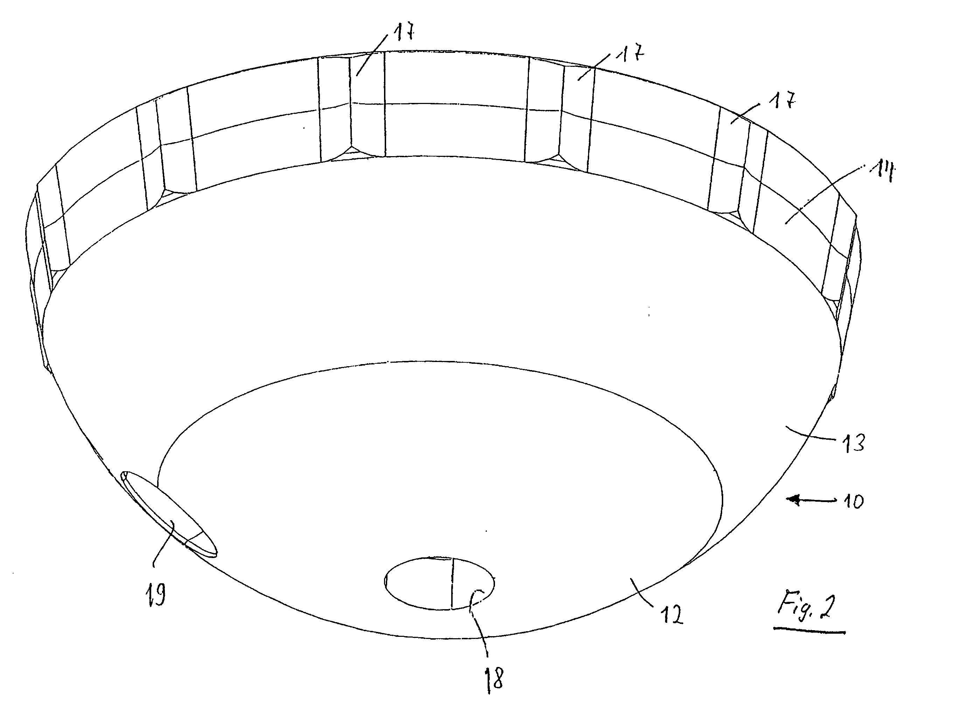

Die Pfanne gemäß Fig. 2 zeichnet sich noch dadurch aus, daß die äquatoriale Kugelsegmentfläche 14 polar gerichtete Rippen 17 umfaßt. Die Rippen 17 sind gleichmäßig über den Umfang verteilt angeordnet und erhöhen die Rotationsstabilität der Pfanne im natürlichen Pfannenlager. Auch tragen sie zusätzlicn zu einem erhöhten Preßsitz im natürlichen Pfannenlager bei.

Bei der Ausführungsform nach Fig. 2 ist im Pol eine Durchgangsbohrung 18 vorgesehen zur Aufnahme einer Knochenschraube. Auch im Übergangsbereich zwischen der polaren und medialen Kugelsegmentfläche ist eine Durchgangsbohrung 19 ausgebildet. Bei Bedarf können auch zwei Durchgangsbohrungen 19 in vorbestimmtem Winkelabstand voneinander vorgesehen sein zur Aufnahme von Knochenschrauben. Die Durchgangsbohrungen 18, 19 können auch als Langlöcher ausgebildet sein, um einen geeigneten Ort für die Knochenschraube innerhalb der durch die Langlöcher bestimmten Grenzen auswählen zu können. Dies gilt insbesondere für die im medialen Bereich vorgesehenen Durchgangsöffnungen. Werden diese als Langlöcher ausgebildet, erstrecken sie sich vorzugsweise in Richtung eine Längengrads.

### Bezugszeichenliste

- 10: Pfanne
- 11: Außenfläche
- 12: Kugelsegmentfläche
- 13: Kugelsegmentfläche
- 14: Kugelsegmentfläche
- 15: Rotationsachse
- 16: Halbkreisbogen
- 17: Rippe
- 18: Durchgangsbohrung
- 19: Durchgangsbohrung

## Patentansprüche

1. Metallene Pfanne für eine Gelenkendoprothese, deren Außenfläche (11) wenigstens drei sich über den Umfang erstreckende konvex nach außen gewölbte Teilflächen (12, 13, 14) mit jeweils unterschiedlichem Radius (R1, R2, R3) umfaßt,
**dadurch gekennzeichnet, daß**
die konvex nach außen gewölbten Teilflächen ausschließlich Kugelsegmentflächen sind, deren Zentren (Z1, Z2, Z3) auf einer gemeinsamen Rotationsachse (15) liegen, wobei der Radius (R3) der medialen Kugelsegmentfläche (13) kleiner ist als der Radius (R2) der polaren Kugelsegmentfläche (12), aber größer ist als der Radius (R1) der äquatorialen Kugelsegmentfläche (14).

2. Pfanne nach Anspruch 1,
**dadurch gekennzeichnet, daß**
das Zentrum (Z1) der peripheren bzw. äquatorialen Kugelsegmentfläche (14) näher am Pol der Pfanne (10) liegt als das Zentrum (Z2) der polaren Kugelsegmentfläche (12).

3. Pfanne nach einem der Ansprüche 1 bis 2,
**dadurch gekennzeichnet, daß**
die äquatoriale Kugelsegmentfläche (14) polar gerichtete Rippen (17) oder dergleichen Erhebungen umfaßt.

4. Pfanne nach einem der Ansprüche 1 bis 3,
**dadurch gekennzeichnet, daß**
die äquatoriale (14) und polare (12) Kugelsegmentflächen jeweils eine geringere Breite aufweisen als die dazwischen liegende bzw. mediale Kugelsegmentfläche (n) (13), insbesondere etwa die halbe Breite der medialen Kugelsegmentfläche (n) (13).

## Claims

1. A metallic socket intended for an articular endoprosthesis, the outer surface (11) of which comprises at least three convexly outwardly bulbed partial surfaces (12, 13, 14) extending over the circumference and each having a different radius (R1, R2, R3),
**characterized in that**
said convexly outwardly bulbed partial surfaces are exclusively ball segment surfaces, the centers (Z1, Z2, Z3) of which are situated on a common rotation axis (15), the radius (R3) of the medial ball segment surface (13) being smaller than the radius (R2) of the polar ball segment surface (12) but larger than the radius (R1) of the equatorial ball segment surface (14).

2. The socket according to claim 1,
**characterized in that**
the center (Z1) of the peripheral or equatorial ball segment surface (14) is situated closer to the pole of the socket (10) than the center (Z2) of the polar ball segment surface (12).

3. The socket according to any one of claim 1 or 2,
**characterized in that**
the equatorial ball segment surface (14) comprises ribs (17) or similar elevations oriented towards the polar direction.

4. The socket according to any one of claims 1 through 3,
**characterized in that**
the equatorial (14) and polar (12) ball segment surfaces each have a smaller width than the medial ball segment surface (n) (13) or ball segment surface (n) (13) situated in between, in particular about half the width of the medial ball segment surface (n) (13).

## Revendications

1. Cotyle métallique pour une endoprothèse articulaire dont la surface extérieure (11) comprend au moins trois surfaces partielles (12, 13, 14) convexes, s'étendant sur le périmètre de manière courbée vers l'extérieur, de rayons différents (R1, R2, R3),
**caractérisé en ce que**
les surfaces partielles convexes courbées vers l'extérieur sont exclusivement des surfaces de segments sphériques, dont les centres (Z1, Z2, Z3) reposent sur un axe de rotation (15) commun, où le rayon (R3) de la surface de segment sphérique médial (13) est plus petit que le rayon (R2) de la surface de segment sphérique polaire (12) mais plus grand que le rayon (R1) de la surface de segment sphérique équatorial (14).

2. Cotyle selon la revendication 1,
**caractérisé en ce que**
le centre (Z1) de la surface de segment sphérique périphérique, respectivement, équatorial, (14) est plus proche du pôle du cotyle (10) que le centre (Z2) de la surface de segment sphérique polaire (12).

3. Cotyle selon l'une des revendications 1 à 2,
**caractérisé en ce que**
la surface de segment sphérique équatorial (14) comprend des nervures (17) ou des surélévations similaires orientées vers la direction polaire.

4. Cotyle selon l'une des revendications 1 à 3,
**caractérisé en ce que**
les surfaces de segment sphérique équatorial (14) et polaire (12) présentent chacune une largeur inférieure à la surface de segment sphérique situé entre, respectivement, médial (n) (13), en particulier approximativement la moitié de la largeur de la surface de segment sphérique médial (n) (13).
